# EUROPEAN PATENT APPLICATION

(11) **EP 2 020 446 A1**
(43) Date of publication of application: **04.02.2009**
(21) Application number: 07301286.6
(22) Date of filing: 31.07.2007
(51) Int. Cl.: C12Q 1/68

(54) **Compositions and methods for detecting histamine related disorders**

(71) Applicant: Vincent, Denis, 30900 Nimes (FR)
(72) Inventor: Vincent, Denis, 30900 Nimes (FR)
(74) Representative: Becker, Philippe

(57) **Abstract**

The present invention generally relates to methods and compositions (e.g., assay kit) for the diagnosis of histamine related disorders. The invention also relates to a novel molecular target of histamine related disorders and the uses thereof for detecting or diagnosing such diseases, as well as to develop adapted and efficient therapeutic treatment thereof. The invention may be used in any mammalian subject, particularly human subjects.

## Description

### FIELD OF THE INVENTION

The present invention generally relates to methods and compositions (e.g., assay kit) for the diagnosis of histamine related disorders. The invention also relates to a novel molecular target of histamine related disorders and the uses thereof for detecting or diagnosing such diseases, as well as to develop adapted and efficient therapeutic treatment thereof. The invention may be used in any mammalian subject, particularly human subjects.

### BACKGROUND OF THE INVENTION

Histamine related disorders represent a group of diseases associated to the metabolism of histamine having essentially no known etiologies. Such group of diseases includes, without limitation, chronic urticaria, angioedema, asthma, and pseudo allergic reactions, including drug reactions.

Chronic urticaria (CU) is defined as recurrent hives occurring for at least 6 weeks. In the majority of cases, from 80 to 20% according to published literature, there is no etiology, despite extensive search.

Angioedema (AE) is defined as recurrent mucosal oedema. In the majority of cases, its etiology is genetically demonstrated, with alteration in the C1 inhibitor enzyme activity. Some patients have no known etiology, and are clinically improved along histamine-free diet. Theses patients have frequently the association of oedema and urticaria lesions. Their disease is called histamine-dependent AE (HDAE).

Pseudo allergic reactions (PAR) are clinically defined as allergic diseases but no allergen can be demonstrated. Histamine-free diet prevents recurrence of these reactions, which are thus called histamine-dependent PAR (HDPAR).

CU patients have either auto-immune, or physical, or histamine dependent CU. This last subset of these CU patients is defined by the presence of urticaria when dietary histamine load is high. This dietary histamine intake is evaluated with dietary questionnaires and is the only method, in association to testing histamine-free diet, to diagnose this type of CU. Suppressing histamine dietary intake is associated with heeling of CU whereas anti-H1 and/or anti-leukotrienes have poor efficacy. It appears that histamine clearance in the body is central to this type of CU. Today, we do not know the histamine intake threshold associated with clinical symptoms. Its physiopathology is also not known. Histamine-free diet is difficult to follow and exposes to a great risk of dietary carences. This type of CU is called histamine-dependent CU (HDCU).

HDPAR and HDAE with no known etiologies are also clinically improved when an histamine free diet is suggested to the patient. But, their physiopathology is also not known.

Theses categories of diseases have not been investigated so far since their physiopathology is not known. Accordingly, there is a need in the art for *in vitro* and *in vivo* assays and methods capable of evaluating histamine metabolism which will recognise histamine dependent CU, or AE or PAR patients from patients with other forms of CU, AE or allergic reaction. There is a need in the art for efficient methods of detecting histamine related disorders, such as CU, AE and PAR, thereby facilitating their suitable treatment and/or prevention.

### SUMMARY OF THE INVENTION

The invention provides novel methods and compositions for detecting histamine related disorders. The invention stems from the unexpected discovery that Diamine oxidase ("DAO") is a biomarker of such disorders, and may be used as a molecular target for monitoring these conditions.

A particular object of the present invention thus relates to a method for the diagnosis of histamine related disorders, such as chronic urticaria, angioedema and pseudo allergic reactions, based on a detection of DAO expression or activity.

A further object of this invention resides in methods for detecting the presence, stage or type of a histamine related disorder in a subject, the method comprising detecting in vitro or ex vivo the presence of an altered DAO expression or activity in a sample from the subject, the presence of such an altered DAO expression or activity being indicative of the presence, stage or type of a histamine related disorder in said subject.

According to specific embodiments of the method, the altered expression is a reduced DAO expression or activity in said sample.

DAO expression or activity may be detected by any technique known per se in the art, such as by sequencing, selective hybridization, selective amplification and/or specific ligand binding. In a particular embodiment, the invention uses reagents and/or techniques allowing the detection (and quantification) of DAO proteins in a biological sample from a subject, or of DAO RNA, or of anti-DAO antibodies (e.g., auto-immune antibodies) in such sample.

In a particular embodiment, this invention resides in methods for detecting the presence, stage or type of a histamine related disorder in a subject, the method comprising determining in vitro or ex vivo the DAO activity in a sample from the subject, comparing such measured activity to a reference or mean value, wherein a decrease in such activity is indicative of the presence of such a disease.

In an other particular embodiment, this invention resides in methods for detecting the presence, stage or type of a histamine related disorder in a subject, the method comprising determining in vitro or ex vivo the level of DAO gene expression in a sample from the subject, comparing such measured level to a reference or mean value, wherein a decrease in such level is indicative of the presence of such a disease.

In an other particular embodiment, this invention resides in methods for detecting the presence, stage or type of a histamine related disorder in a subject, the method comprising determining in vitro or ex vivo the DAO gene polymorphism in a sample from the subject, wherein an increased polymorphism as compared to a reference value or condition is indicative of the presence of such a disease.

In an other particular embodiment, this invention resides in methods for detecting the presence, stage or type of a histamine related disorder in a subject, the method comprising determining in vitro or ex vivo the (relative) amount of DAO protein in a sample from the subject, comparing such measured amount to a reference or mean value, wherein a decrease in such amount is indicative of the presence of such a disease.

In an other particular embodiment, this invention resides in methods for detecting the presence, stage or type of a histamine related disorder in a subject, the method comprising determining in vitro or ex vivo the presence of anti-DAO antibodies in a sample from the subject, such a presence being indicative of the presence of such a disease.

The biological sample may be any sample containing DAO gene or polypeptides or antibodies. Such samples typically include tissue samples, biopsies, biological fluids, etc. In a typical embodiment, the fluid sample derives (e.g., by dilution, concentration, purification, separation, etc.) from total blood, serum, plasma, urine, mucosa, in particular serum, digestive fluid or bronchial fluid. In an other particular aspect, the sample comprises cells substantially purified from mucosal biopsies of a donor, such as digestive and respiratory mucosa.

A further object of this invention is a method of assessing the efficacy of a treatment of a histamine related disorder in a subject, the method comprising comparing (in vitro or ex vivo) DAO expression or activity in a sample from the subject prior to and after said treatment, an increased expression being indicative of a positive response to treatment.

The invention also relates to the use of a DAO protein or of a nucleic acid encoding a DAO protein in a method of detecting histamine-related disorders.

The invention also relates to the use of DAO-specific ligands and/or DAO-specific nucleic acid primers or probes, in a method of detecting histamine-related disorders.

The invention also concerns the use of a compound or treatment that increases DAO expression or activity in a subject, for the manufacture of a pharmaceutical composition for treating histamine related disorders, as well as corresponding method of treatment.

A further aspect of this invention resides in the use of a compound that stimulates DAO gene expression for the manufacture of a pharmaceutical composition for treating histamine related disorders, as well as in a corresponding method of treatment.

The invention also resides in methods of treating histamine related disorders in a subject in need thereof, comprising administering to said subject an effective amount of a compound as defined above. In a preferred embodiment, the subject has a reduced DAO expression or activity.

The invention further relates to methods of selecting biologically active compounds on histamine related disorders, the method comprising a step of selecting compounds that mimic or stimulate DAO expression or activity.

In a specific embodiment, the method comprises contacting a test compound with a recombinant host cell comprising a reporter construct, said reporter construct comprising a reporter gene under the control of a DAO gene promoter, and selecting the test compounds that stimulate expression of the reporter gene.

A further aspect of this invention resides in a nucleic acid primer that allows (specific) amplification of a DAO RNA.

A further aspect of this invention resides in a nucleic acid probe that (specifically) hybridizes to a DAO RNA.

A further aspect of this invention resides in an antibody (including derivatives thereof and producing hybridomas), that (specifically) binds a DAO protein.

The invention further relates to kits comprising a primer, probe or antibody as defined above. Such kits may comprise a container or support, and/or reagents to perform an amplification, hybridization or binding reaction.

### LEGEND TO THE FIGURES

**Figure 1****.** Regional distribution in the gastrointestinal tract (fund : fundus; ant : antrum; D1 : proximal duodenum; D2 : intermediate duodenum) from patients biopsies with CIU(n=24) and controls (n=6). of (A ) MAO-A activity, (B) MAO-B activity, (C) Histamine (*D1: P value equals 0.0183; *D2: P value equals 0.0077) and (D) DAO activity Histamine (*D1: P value equals 0.0012; *D2: P value equals 0.0001) (E) Serum DAO activity from patients with CIU (n=18) and controls (n=5). P value is less than 0.0001.

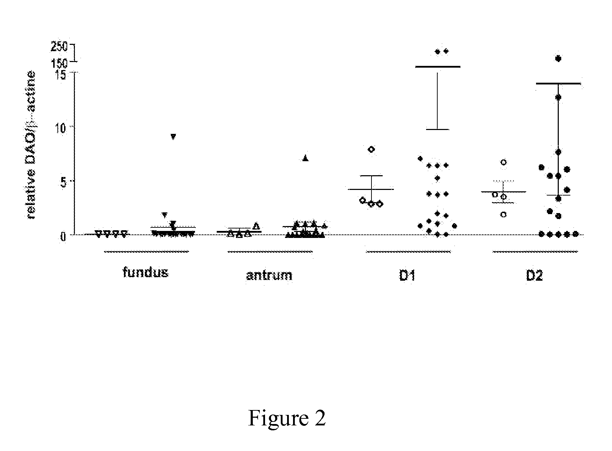

**Figure 2****.** DAO expression normalized with bactin as an internal control by real time SYBR green PCR (Light Cycler II). Open triangle, rhombus and circle are normal patients, filled one are patients with CIU.

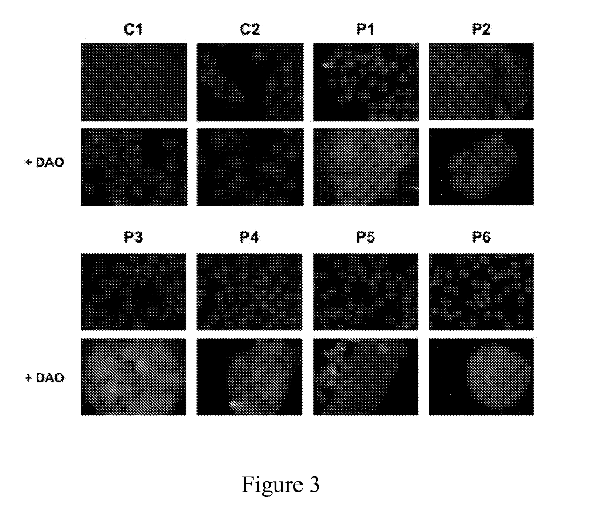

**Figure 3****.** Serum reactivity tested by immunofluorescence. For each group of patient upper line are HaCat cell line, bottom line labeled +DAO are DAO-13 HaCat. Cx represent control patients and Px patient with CIU. All patients' sera were used at a dilution of 1:100.

### DETAILED DESCRIPTION OF THE INVENTION

This invention generally relates to methods of diagnosing histamine related disorders, as well as to methods of developing improved therapies for such conditions, using DAO as a molecular target.

DAO: Diamine oxidase (also known as Amiloride Binding Protein 1) is an enzyme involved in the metabolism of food histamine. DAO is highly expressed all along the small intestine mucosa (by the enterocytes), where nutriments, and food histamine, are captured by the organism. Its highest activity is in the intestinal mucosa where it is localized in the cytoplasm of the mature enterocytes of the small and large bowel. The nucleic acid and amino acid sequences of human DAO have been cloned and reported in the literature. These sequences are available e.g., under accession number NM001091, or in Barbry et al, PNAS 87 (1990) 7347. Within the context of this invention, the term DAO gene designates any nucleic acid (e.g., genomic DNA, cDNA, RNA, etc.) comprising the sequence of all or a portion of a human gene encoding a DAO protein, as defined above. This includes any naturally-occurring variants, such as splicing isoforms, polymorphisms, truncated variants, etc., as well as synthetic analogs thereof. The term DAO protein designates any polypeptide comprising an amino acid sequence encoded by a DAO gene as defined above, as well as fragments thereof, particularly immunologically-reactive fragments thereof.

Within the context of this invention, the terms "diagnosing" or "diagnosis" include, generally, methods of detecting, assessing, predicting, monitoring or classifying a disease condition.

The term "reduced" when referring to a DAO expression or activity designates a reduction in the level of expression or activity of at least 10%, as compared to a reference or mean value, more preferably a reduction of at least 20, 25, 30, 35, 40 % or more.

As discussed above, the invention relates to methods of diagnosing histamine related disorders comprising a step of assessing the DAO expression or activity in a sample from a subject, wherein a reduced expression or activity is indicative of the presence of such a disorder.

Preferred methods include the steps of (a) providing cells or fluid from a biological sample from an individual being diagnosed (b) measuring DAO activity, detecting DAO expression and/or DAO polymorphism on the cells, (c) comparing with negative sample from an individual who does not have such a condition, or with a reference value (previously) determined as an average or mean value from various control subjects, and/or (d) testing for the presence of auto-immune antibodies directed against the protein sequence of DAO.

Low DAO activity, presence of anti-DAO antibody, polymorphism of DAO gene, or decreased DAO gene expression indicates that the patient has histamine-dependant CU, PAR or AE.

In one aspect of this embodiment, the test biological sample is a fluid sample, including, but not limited to, serum, mucosal such as digestive and bronchial fluid. In one aspect, the isolated cells are provided as substantially purified cells from mucosal biopsies of a donor, such as digestive and respiratory mucosa. In another aspect, the isolated cells are provided as a cell line, including cell lines over-expressing DAO under the control of a constitutive or controlled promoter. In another aspect, the isolated cells are provided from a control donor who has no disease.

In one aspect of this embodiment of the invention, DAO gene polymorphism, level of expression of DAO mRNA and protein are addressed in fluid sample and/or in isolated cells or tissues.

In another aspect of this embodiment of the invention, the method additionally includes testing the individual DAO activity, assayed in fluid sample and/or in isolated cells and/or in mucosal biopsies.

In one aspect of this embodiment of the invention, cell lines over-expressing DAO, and the method further includes detecting expression of DAO by these cells. Increased detection of DAO on these cells, in the presence of the biological sample of patients, as compared to level of detection when contacted with a negative control sample from an individual who does not have chronic urticaria, AE or PAR, further indicates that the patient has anti-DAO antibodies and therefore HD chronic urticaria, HDAE or HDPAR.

In another aspect of this embodiment of the invention, the step of testing the biological sample is performed by flow cytometry and/or ELISA. In one aspect the step of detecting is performed using antigen-binding fragment of DAO combined with theses techniques.

Various techniques known in the art may be used to detect or quantify altered DAO expression, including sequencing, hybridisation, amplification and/or binding to specific ligands (such as antibodies). Other suitable methods include allele-specific oligonucleotide (ASO), allele-specific amplification, Southern blot (for DNAs), Northern blot (for RNAs), single-stranded conformation analysis (SSCA), PFGE, fluorescent in situ hybridization (FISH), gel migration, clamped denaturing gel electrophoresis, heteroduplex analysis, RNase protection, chemical mismatch cleavage, ELISA, radio-immunoassays (RIA) and immuno-enzymatic assays (IEMA).

Amplification may be performed according to various techniques known in the art, such as by polymerase chain reaction (PCR), ligase chain reaction (LCR), strand displacement amplification (SDA) and nucleic acid sequence based amplification (NASBA). These techniques can be performed using commercially available reagents and protocols. Preferred techniques use allele-specific PCR or PCR-SSCP. Amplification usually requires the use of specific nucleic acid primers, to initiate the reaction.

Nucleic acid primers useful for amplifying sequences from the DAO gene or RNA are complementary to and specifically hybridize with a portion of the DAO gene or RNA. Typical primers of this invention are single-stranded nucleic acid molecules of about 5 to 60 nucleotides in length, more preferably of about 8 to about 25 nucleotides in length. Perfect complementarity is preferred, to ensure high specificity. However, certain mismatch may be tolerated. Specific examples of such primers are disclosed in the experimental section.

The invention also concerns the use of a nucleic acid primer or a pair of nucleic acid primers as described above in a method of detecting the presence of or predisposition to histamine related disorder in a subject.

In another embodiment, detection is carried out by a technique using selective hybridization. A particular detection technique involves the use of a nucleic acid probe specific for DAO gene or RNA, followed by the detection of the presence and/or (relative) amount of a hybrid. The probe may be in suspension or immobilized on a substrate or support (as in nucleic acid array or chips technologies). The probe is typically labeled to facilitate detection of hybrids.

In this regard, a particular embodiment of this invention comprises contacting the sample from the subject with a nucleic acid probe specific for DAO, and assessing the formation of a hybrid. Within the context of this invention, a probe refers to a polynucleotide sequence which is complementary to and capable of specific hybridisation with a (target portion of a) DAO gene or RNA, and which is suitable for detecting the presence or (relative) amount thereof in a sample. Probes are preferably perfectly complementary to a sequence of the DAO gene or RNA. Probes typically comprise single-stranded nucleic acids of between 8 to 1000 nucleotides in length, for instance of between 10 and 800, more preferably of between 15 and 700, typically of between 20 and 500. It should be understood that longer probes may be used as well.

Specificity indicates that hybridisation to the target sequence generates a specific signal which can be distinguished from the signal generated through non-specific hybridisation. Perfectly complementary sequences are preferred to design probes according to this invention. It should be understood, however, that a certain degree of mismatch may be tolerated, as long as the specific signal may be distinguished from non-specific hybridisation.

The invention also relates to a nucleic acid chip comprising a probe as defined above. Such chips may be produced in situ or by depositing clones, by technique known in the art, and typically comprise an array of nucleic acids displayed on a matrix, such as a (glass, polymer, metal, etc.) slide.

Alteration in the DAO expression may also be detected by detecting the (relative) amount of a DAO polypeptide. In this regard, a specific embodiment of this invention comprises contacting the sample (which may comprise biological fluids such as blood, plasma, serum, etc.) with a ligand specific for a DAO polypeptide, and determining the formation of a complex.

Different types of ligands may be used, such as specific antibodies. In a specific embodiment, the sample is contacted with an antibody specific for a DAO polypeptide, and the formation of an immune complex is determined. Various methods for detecting an immune complex can be used, such as ELISA, radioimmunoassays (RIA) and immuno-enzymatic assays (IEMA).

Within the context of this invention, an antibody designates a polyclonal antibody, a monoclonal antibody, as well as any fragments or derivatives thereof having substantially the same antigen specificity. Fragments include Fab, Fab'2, CDR regions, etc. Derivatives include single-chain antibodies, humanized antibodies, poly-functional antibodies, etc.

In a specific embodiment, the method comprises contacting a sample from the subject with (a support coated with) an antibody specific for a DAO polypeptide, and determining the presence of an immune complex.

Alteration in the DAO activity may also be detected by detecting the presence or (relative) amount of anti-DAO antibodies (e.g., autoimmune antibodies) in the sample from the subject. Indeed, the inventor has shown that the reduced DAO activity may be due to the presence in the serum of patients of autoimmune antibodies directed against DAO.

In this regard, a specific embodiment of this invention comprises contacting the sample (which may comprise biological fluids such as blood, plasma, serum, etc.) with a DAO polypeptide or an immunologically reactive fragment thereof (i.e., a fragment comprising at least a DAO epitope), and determining the formation of a complex.

The diagnostic methods of the present invention can be performed in vitro, ex vivo or in vivo, preferably in vitro or ex vivo. They use a sample from the subject, to assess any alteration in DAO expression or activity. The sample may be collected according to conventional techniques and used directly for diagnosis or stored. The sample may be treated prior to performing the method, in order to ensure or improve availability of nucleic acids or polypeptides for testing. Treatments include, for instance, lysis (e.g., mechanical, physical, chemical, etc.), centrifugation, etc. Also, the nucleic acids and/or polypeptides may be pre-purified or enriched by conventional techniques, and/or reduced in complexity. Nucleic acids and polypeptides may also be treated with enzymes or other chemical or physical treatments to produce fragments thereof. Considering the high sensitivity of the claimed methods, very few amounts of sample are sufficient to perform the assay.

The assay may be performed in any suitable device, such as a plate, tube, well, glass, etc. In specific embodiments, the contacting is performed on a substrate coated with the reagent, such as a nucleic acid array or a specific ligand array. The substrate may be a solid or semi-solid substrate such as any support comprising glass, plastic, nylon, paper, metal, polymers and the like. The substrate may be of various forms and sizes, such as a slide, a membrane, a bead, a column, a gel, etc. The contacting may be made under any condition suitable for a complex to be formed between the reagent and the nucleic acids or polypeptides of the sample.

Another embodiment of the present invention relates to a method to treat CU, PAR or AE, comprising administering to the individual a pharmaceutical composition that increases the expression or the activity of DAO.

This invention generally relates to methods, reagents and kits that are useful for the detection of HD chronic urticaria, HD AE and HD PAR. This invention can be used to identify patients that have chronic urticaria, AE and PAR related to an abnormal DAO activity, to further understand the mechanisms of chronic urticaria, AE and PAR and, in addition, to allow the identification of therapeutic target for the treatment of chronic urticaria, AE and PAR.

Further aspects and advantages of this invention will be disclosed in the following examples, which shall be regarded as illustrative and do not limit the scope of the present application.

### EXAMPLES

### A - Materials and Methods

### Determination of Monoamine Oxidase (MAO) A and B Activity.

Activity in the homogenates from the biopsies were measured as described elsewhere (Muller et al., 1997). Briefly, the resuspended pellets were incubated for 6 min with 14C-5-hydroxytryptamine (MAO A assay) or 14C-beta-penylethylamine (MAO B) used as substrates. MAO activity. Samples were incubated at 37°C for 20 min in phosphate buffer with increasing concentrations of 5-[14C]HT (0-500 µM) or beta -[14C]PEA (0-10 µM) to measure MAO-A and MAO-B activities, respectively. The irreversible MAO inhibitor pargyline (10 µM) was used to defined nonspecific MAO-A or MAO-B activity. Reaction was ended by adding 4 N HCl at 4°C.

### Histamine dosage.

Histamine concentrations were measured using a commercially available histamine RIA kit (IM1659 Beckman-Coulter, Miami, Florida, USA)

### Determination of Diamine Oxidase Activity (DAO).

The procedure used was the method of Kusche et al. (1975). The assay mixture for the test consisted of 0.6 ml of 0.1 M sodium phosphate buffer (pH 7.6), 0.1 ml of test sample solution, 3) 0.05 ml of substrate solution (containing 4.5 mM putrescine dihydrochloride and 1.0 µCi/ml of [1,4-14C]putrescine dihydrochloride). Incubation period of 10 min at 37°C at which time the reaction was stopped by adding 1.0 ml of alkaline buffer (800 mM NaOH, 600 mM NaHCO3 at pH 12.2). The labeled reaction product ([C14]-Delta 1-pyrroline) was directly extracted into 6 ml of scintillation fluid (toluene containing 3.5 g/liter of 2,5-diphenyloxazole).

### PCR.

RNA was extracted from samples using RNeasy Mini Kit (Quiagen) using 5 mg of tissues. Amplification by RT-PCR : expression of MAO, DAO and b-actin was first determined by semi-quantitative mRNA analysis. OneStep RT-PCR kit (Quiagen) was used to synthesize cDNA and PCR reactions were performed using a MWG primus 96 (MWG Biotech United Kigdom, Ltd). Primers sequence are as follows :
MAO-A and MAO-B : 5'-GGACAGGAGAGGAAATTTGTGGGCGGAT-3' (SEQ ID
NO: 1) and 5'-GCCTGGCACGGAGGGCAAATGTCTC-3' (SEQ ID NO: 2) ;
b-actin : 5'-GGCATCGTGATGGACTCCG-3' (SEQ ID NO: 3) and
GCTGGAAGGTGGACAGCG-3' (SEQ ID NO: 4) ; and
DAO : 5'-AAGGCAGGGGTGTTTTCAGA-3' (SEQ ID NO: 5) and 5'-
TGAAGTTGTCC GCAGCACCA-3' (SEQ ID NO: 6).

For quantitative real-time PCR, one strand reverse transcription was performed using two µg of RNA and Promega reverse transcription system (Promega), 20 µl of RT mixture contained: 1 x RT-buffer (10 mM Tris-HCl pH 9.0, 50 mM KCl, 0.1% Triton X-100), 1 mM of each dNTP, 1 U/µl recombinant RNAsin Ribonuclease inhibitor, 15 U of AMV reverse transcriptase, 0.5 µg of random primers. The cDNA reaction was incubated for 10 min at 25 °C, followed by 60 min at 37 °C and heated 15 min at 70 °C. Primer design and light cycler amplification : the primer pairs for DAO and b-actin were selected with the assistance of Oligo 6 primer. The primer sequences used in this study were:
Oligo sense DAO : 5'-TCAAAAGGAAGCTGCCTAAGT-3' (SEQ ID NO: 7) and Oligo antisense DAO : 5'-GGGTCGTTCTGGTGGTAGAT-3' (SEQ ID NO: 8) ; Oligo sense b-actin : 5'-AGCACGGCATCGTCACCAACT-3' (SEQ ID NO: 9) and Oligo antisense b-actin: 5'-TGGCTGGGGTGTTGAAGGTCT-3' (SEQ ID NO: 10). Amplification of the cDNA coding for DAO and b-actin were performed using LightCycler technology (Roche Diagnostics, Basel, Switzerland). The reactions were performed in a volume of 10 µl of a using LC-FastStart DNA Master plus SYBR green I kit (Roche). LightCycler mastermix (8 µl) was filled in the LightCycler glass capillaries and 2 µl cDNA was added as PCR template. The following LightCycler protocol was used 95 °C for 10 s; denaturation, 60°C for 5 s; annealing, 72 °C for 5 s; elongation, 95 °C for 5 s.

### Plasmid construction.

Wild type human growth DAO, in a cloning vector, was a generous gift of Dr. Hubert Schwelberger (Labor für Theoretische Chirurgie, Universitätsklinik für Chirurgie, Universität Innsbruck). The cDNA fully comprised between BahmI and EcoRI restriction site of the vector was cloned in mammalian expression vector pcDNA3.1 (Clontech Laboratories, Palo Alto, CA).

### Cell Culture and Transfection.

Human keratinocyte cell line (HaCaT) cells were cultured in RPMI-1640 medium (Sigma-Aldrich) supplemented with 10% (v/v) FBS, 1% (v/v) of nonessential amino acids, 2 mM glutamine and streptomycin-penicillin (250 µg/mL-250 IU/ml), at 37°C in a humidified atmosphere with 5% CO₂. Transfection stable....

### Cell staining.

HaCat and D-13 HaCat cells were grown on glass coverslips in normal condition. After one day, cells were washed 3X with PBS and fixed with 4 % paraformaldehyde for 10 min at room temperature. Cells were then rinsed with PBS, permeabilized by PBS containing 0.1% triton x100 for 20 min. The cells were then blocked in PBS containing 3% BSA for 15 min and incubated with patient's serum at various dilution (1:10 to 1:500) in PBS containing 3% BSA at 37°C in a humidified room. Coverslips were then washed 3X and incubated for 1 hour with a Cy2-conjugates anti-Human IgA + IgG + IgM (H + L) Goat antibody (Jackson ImmunoResearch Laboratories, Inc) at 1:200 dilution in PBS containing 3% BSA, for 1 hour in a humidified room at 37°C. Cell nuclei were counterlabelled using DAPI (300 nM, Molecular Probes). Cells were then washed in PBS (3X) and mounted with mowiol. Cy2 and DAPI staining were observed using epifluorescent microscopy (Olympus....) with filter sets for fluorescein (lex=494 nm lem=525 nm) and nuclei observed using filter sets for DAPI (lex=358 nm lem=461 nm). Images were analyzed first with the microscope software and then reconstructed with iView MediaPro 3 (Microsoft) and ImageJ (National Center for Biotechnology Information).

### Statistical analysis.

Values are expressed as means ± SE. The statistical significance of differences among the experimental groups was evaluated by GraphPad Prism.

### B - Results

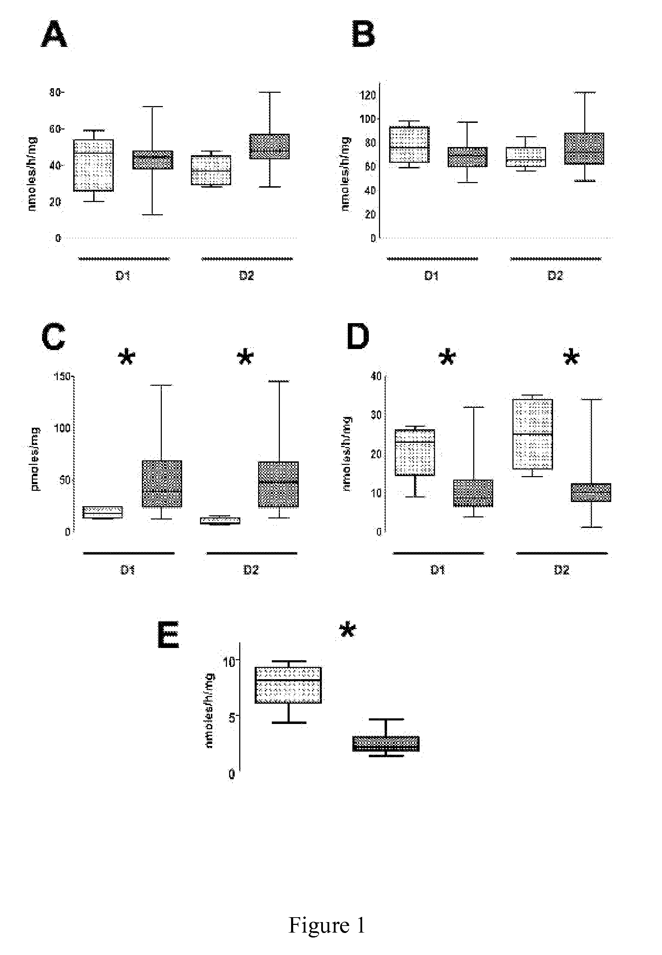

The inventor has discovered that accumulation of food histamine in the stomacal and duodenal mucosa of chronic urticaria patient is correlated with an impairment of DAO activity. MAO activity does not appear to be involved in CU physiopathology, and was used as an internal control. MAOs are widely distributed throughout the body; their concentration is especially high in the liver, kidney, stomach, intestinal wall, and brain. MAOs are currently subclassified into two types, A and B, which differ in their substrate specificity and tissue distribution. No significant variations in the different enzymatic MAO (A and B) activities were found along the fundus (respectively 41.2 and 73 nmoles/h/mg) and antrum (44.8 and 75 nmoles/h/mg) or along the proximal duodenum (42.2 and 77.3 nmoles/h/mg) or the intermediate duodenum (32.7 and 66.8 nmoles/h/mg). Results obtained with our patients showed almost equal levels than in the controls (figure 1 A and B): no significant variation was observed between patients presenting CIU (n=25) or not (control group n=6).

The inventor has discovered that a significant reduction in the histamine concentration was observed by the stomach (fundus: 56.2 pmoles/mg; antrum: 51.6 pmoles/mg) towards the intestine (proximal duodenum: 18 pmoles/mg; intermediate duodenum: 9.8 pmoles/mg ; figure 1 C)) for normal subjects, whereas only a modest reduction in the histamine concentration was observed along the proximal duodenum or along the intermediate duodenum for subjects with CU (fundus: 88.2 pmoles/mg; antrum: 66.8 pmoles/mg; not shown, and proximal duodenum: 49.9 pmoles/mg; intermediate duodenum: 46.6 pmoles/mg; figure 1 C).
The inventor has discovered that histamine content is significantly higher in patients with CU compared to control group. For proximal duodenum P value equals 0.0183 and for intermediate duodenum P value equals 0.0077.

The inventor has discovered that the measured DAO activity was substantially affected in the proximal duodenum (11.7 nmoles/h/mg) or the intermediate duodenum (10.6 nmoles/h/mg) for CIU patients when compared to control group (proximal duodenum: 21.1 nmoles/h/mg; intermediate duodenum: 23.4 nmoles/h/mg) with a P value of 0.0012 and 0.0001, respectively.

The inventor has discovered that diamine oxidase activity in the sera was also much lower among patients (n=18) as compared to control (n=6): patients' average was only 30.4% of control DAO activity.

The inventor has discovered that serum DAO activity is correlated with the intestinal activity. DAO activities in serum, as opposed to biopsies, are sufficient to estimate functional DAO impairment.

The inventor has discovered that accumulation of histamine towards the intestine of CU patients whereas the diamine oxidase is specifically affected and decreased.

DAO is known to be expressed at high level in enterocyte cells of the intestinal epithelium. Thus, we have checked the presence of DAO expression using reverse transcriptase and real time PCR to quantify precisely the amount of DAO in biopsies samples of patient with CIU. MAO and b-actin were also amplified, as internal control. Observed DAO expression levels in fundus and antrum were weak or barely visible amplification. Amplification of DAO was visible for all patients (normal and CIU) duodenum D1 and D2 but difference in the amount of DAO mRNA could count for the strength of its activity.

The inventor has discovered that expression is low both in D1 and D2 is severely impaired in CU patients stressing a possible involvement of the transcriptional regulation of DAO expression in CU.

Diamine oxidase could be inhibited by a variety of compounds. Our experiment consisted of mixing the serum of a healthy patient with, sequentially, those of patients having CU. Indeed serum activity DAO is correlated with the intestinal activity.

The inventor has discovered a strong inhibition of serum DAO activity for HDCU patients (table 1), due to the presence of anti-DAO antibodies.

**Table 1 : Inhibition of DAO activity by the plasma of CU, AE, PAR patients.**

| DAD activity : theorical DAO | | | | |
|---|---|---|---|---|
| | DAO activity | C+Px | activity | Inhibition in % |
| C | 7.4 | | | |
| P1 | 1.5 | 2.3 | 4.4 | 46.2 |
| P2 | 3.1 | 2.2 | 5.3 | 58.1 |
| P3 | 2.4 | 1.4 | 4.8 | 71.4 |
| P4 | 2.7 | 1.7 | 5.0 | 88.2 |
| P5 | 2.1 | 4.1 | 4.8 | 13.7 |
| P6 | 1.9 | 2.0 | 4.7 | 57.0 |
| P7 | 3.0 | 1.5 | 5.2 | 71.1 |
| P8 | 1.8 | 2.3 | 4.6 | 50.1 |
| P9 1.4 | 1.4 | 1.1 | 4.4 | 74.9 |
| Average | | | | 56.7 |
| SE | | | | 12.9 |

D-13 Hacat and cell line overexpression (X50) DAO cDNA as tested by qPCR. Strengh and specifity of signal were tested by using serum between 1:10 and 1/500 and reveled with an anti-Human IgA + IgG + IgM (H + L) Goat antibody. Three group are observed : signal low, closed to control group, signal present both in Hacat cells and D-13 HaCat cells, regardless of the DAO overexpression in those cells..

### References

Kusche J, Lorenz W and Schmidt J (1975) Oxidative deamination of biogenic amines by intestinal amine oxidases: histamine is specifically inactivated by diamine oxidase. Z Phys Chem 356: 1485-1496.

Muller W.E., Rolli M., Schafer C., Hafner U. (1997) Effects of Hypericum extract (LI 160) in biochemical models of antidepressant activity. Pharmacopsychiatry 30: S102-S107.

Haimart M, Launay JM, Zurcher G, Cauet N, Dreux C, Da Prada M. Simultaneous determination of histamine and N alpha-methylhistamine in biological samples by an improved enzymatic single isotope assay. Agents Actions. 1985 Apr;16(3-4):71-5.

## Claims

1. A method for detecting the presence, stage or type of a histamine related disorder in a subject, the method comprising detecting in vitro or ex vivo the presence of an altered DAO expression or activity in a sample from the subject, the presence of such an altered DAO expression or activity being indicative of the presence, stage or type of a histamine related disorder in said subject.

2. The method of claim 1, wherein the altered expression is a reduced DAO expression or activity.

3. The method of claim 1 or 2, wherein the DAO expression or activity is detected by sequencing, selective hybridization, selective amplification and/or specific ligand binding.

4. The method of any one of claims 1 to 3, comprising determining in vitro or ex vivo the DAO activity in a sample from the subject, comparing such measured activity to a reference or mean value, wherein a decrease in such activity is indicative of the presence of such a disease.

5. The method of any one of claims 1 to 3, comprising determining in vitro or ex vivo the level of DAO gene expression in a sample from the subject, comparing such measured level to a reference or mean value, wherein a decrease in such level is indicative of the presence of such a disease.

6. The method of any one of claims 1 to 3, comprising determining in vitro or ex vivo the DAO gene polymorphism in a sample from the subject, wherein an increased polymorphism as compared to a reference value or condition is indicative of the presence of such a disease.

7. The method of any one of claims 1 to 3, comprising determining in vitro or ex vivo the (relative) amount of DAO protein in a sample from the subject, comparing such measured amount to a reference or mean value, wherein a decrease in such amount is indicative of the presence of such a disease.

8. The method of any one of claims 1 to 3, comprising determining in vitro or ex vivo the presence of anti-DAO antibodies in a sample from the subject, such a presence being indicative of the presence of such a disease.

9. The method of any one of the preceding claims, wherein the biological sample is selected from tissue samples, biopsies and biological fluids, preferably from total blood, serum, plasma, urine, mucosa, in particular serum, digestive fluid or bronchial fluid, cells substantially purified from mucosal biopsies, such as digestive and respiratory mucosa.

10. A method of assessing the efficacy of a treatment of a histamine related disorder in a subject, the method comprising comparing (in vitro or ex vivo) DAO expression or activity in a sample from the subject prior to and after said treatment, an increased expression being indicative of a positive response to treatment.

11. A method of selecting biologically active compounds on histamine related disorders, the method comprising a step of selecting compounds that mimic or stimulate DAO expression or activity.

12. The method of any one of the preceding claims, wherein the histamine related disorder is selected from chronic urticaria, angioedema, asthma, and pseudo allergic reactions, including drug reactions.

13. A kit comprising a primer, probe or antibody specific for a DAO gene or protein ; or a DAO protein or an immunologically reactive fragment thereof ; as well as a container or support, and/or reagents to perform an amplification, hybridization or binding reaction.
